# EUROPEAN PATENT SPECIFICATION

(11) **EP 0 437 179 B1**
(45) Date of publication and mention of the grant of the patent: **01.04.1998**
(21) Application number: 90810959.8
(22) Date of filing: 06.12.1990
(51) Int. Cl.: C11D 7/10, C11D 7/16, C11D 3/04, C11D 3/06, C11D 17/04, A61L 2/00

(54) **PH-controlled protein removing cleaner**
PH-geregeltes Reinigen zur Entfernung von Protein
Nettoyant à pH contrôlé pour enlever des protéines

(30) Priority: 15.12.1989 US 451488
(43) Date of publication of application: 17.07.1991
(73) Proprietor: Novartis AG, 4058 Basel (CH)
(72) Inventor: Tsao, Fu-Pao, Dr., Lawrenceville, GA 30245 (US); Dandridge, Rosalind, Doraville, GA 30360 (US)

(56) References cited:
- EP-A- 0 076 136
- EP-A- 0 124 461
- EP-A- 0 139 994
- EP-A- 0 142 642
- EP-A- 0 349 487
- WO-A-86/05695
- GB-A- 2 169 508
- US-A- 4 127 423
- US-A- 4 490 389

## Description

The invention relates to the field of cleaning compositions, more specifically to the use of cleaning compositions for the removal of protein from contact lenses.

Cleaning compositions of various types have been known for many years, comprising many different components. Such compositions are used to solubilize, abrade, oxidize, or reduce surfaces in an effort to remove undesirable surface adherents and restore a desired surface. Each type of surface to be cleaned presents its own characteristics which must be taken into account when choosing an appropriate cleaning composition therefor. Polymeric substrates offer particularly problematic issues in choice of cleanser composition due to the relatively large amounts of proteins which adhere thereto.

Especially thorny cleaning problems arise when the substrate to be cleaned is a contact lens. In addition to the parameters to be considered for the polymeric material of the contact lens per se, one must also take into account that the optical surfaces cannot be scarred, residue must be quickly and easily rinsed away with very small volumes of fluid, tonicity must be in an appropriate range so as not to destabilize the material, and the cleaning system has to be non-toxic and non-allergenic (at least to the extent residue remains or is likely to remain).

Specifically with reference to contact lens cleaning, protein adhesion and build up on the lens, sequestered primarily from tear fluid, has been a persistant and nagging problem. To deal with this difficulty, three general approaches have been attempted. The earliest attempt was to include an abrasive component, so that upon rubbing the lens therewith, the adhering protein would be broken up and town away from the lens. Compositions of this type include slurries of organic compounds such as sucrose or dextrose, of inorganic compounds such as silica, sodium chloride, aluminium oxide or sodium carbonate, of polymeric materials such as nylon beads or silicon polymer beads, or of other particulate matter such as glass beads. A typical representative of this first type is US-A-4,127,423.

A second type of attempted solution involved adding a surfactant. The surfactant is added to substantially solubilize the adherent protein. Compositions of this type include siloxane surfactants, alkylglycosides or polyalkyleneoxy modified silicone. Attempts were also made in the combination of the two types above. Such compositions include Opticlean™ and Restore™.

A third type of attempt to deal with the adherent protein has been to utilize an enzyme to digest the protein.

A fourth type of attempt to deal with the adherent protein is the use of hydrogen peroxide or a precursor thereof in aqueous solutions as disclosed e.g. in EP-A-124 461, WO-A-86/05695 or EP-A-139 994. The hydrogen peroxide in such systems is always reduced to water in a second step.

EP-A-349 487 describes an antimicrobial ophthalmic solution containing Bradosol® and methods of using the same.

Ophthalmic solutions containing hydroxyethyl cellulose are described in GB-A-2 169 508, which solutions may serve as artificial tears.

EP-A-76 136 describes aqueous disinfecting solutions for contact lenses, which solutions contain certain quaternary ammonium salts, e.g. Onamer M®.

US-A-4 490 389 describes a method for sterilizing contact lenses in an aqueous solution, wherein said solution has a pH between about 6 and 9 and comprises an ene-diol compound and copper ions.

Of course the protein method can be used alone or in combination with any of the aforementioned methods of dealing with protein deposits. Typical available enzymatic cleaners for contact lenses include papain, subtilisin, pancreatin and other proteases.

While each of these methods has been somewhat successful in protein removal, each presents its share of drawbacks. For example, many abrasives are too harsh for use with contact lenses. Surfactants, in efficacious amounts for their surfactant properties, can irritate the eye terribly. Enzymes which degrade protein must never be placed into the eye for obvious reasons. Furthermore, the smaller protein fragments which result may be taken up further by the lens material or penetrate deeper into the lens matrix. In addition, enzymes if injected into the environment in substantial quantities create a considerable environmental hazard.

One object of the present invention is related to the use of a cleaner composition for removal of protein from a contact lens which avoids the problems of the art as noted above.

Another object of the invention is related to the use of a simple, yet effective cleaning regimen for contact lenses.

Still another object of the invention is related to the use of a means for creating a cleaning solution in accordance with the preceeding objectives in situ by the user thereof.

Yet another object is related to the use of an environmentally responsible formulation for removing protein from contact lenses.

Surprisingly, these and other objectives of the invention are achieved by a pH controlled cleaning composition consisting of water, a tonicity builder and a pH regulator, in the substantial absence of a protein-digesting enzyme and in the substantial absence of a protein dissolving efficacious amount of a surfactant, and by a method of removing protein therewith from a contact lens having protein or proteinaceous deposits thereon consisting of contacting said contact lens with said composition for a time sufficient to loosen and remove substrate-adhered protein therefrom to result in a cleaned substrate and removing said substrate (absent the formerly adhered protein) from said composition.

The invention is thus directed to the use of a solution having as its only requirements:
(a) water as a carrier;
(b) a tonicity builder in an amount sufficient to render said composition ophthalmically acceptable;
(c) a pH regulating agent in an amount sufficient to provide and maintain an alkaline pH in the range of 7.5 to 11.5 in the presence of the other components of the composition;
(d) substantially no protein digesting enzyme.
Said solution is further characterized by the absence of a protein dissolving effective amount of a surfactant.

The tonicity builder can be any ionic or non-ionic species which is sufficiently soluble to give the appropriate tonicity, which after the cleaning regimen is complete is ocularly acceptable, and which is compatible with the lens material. The appropriate tonicity is preferably approximate isotonicity at the conclusion of the dosage regimen

Typical tonicity builders for use in the invention include suitable water soluble salts compatible with ocular tissue, preferably alkali or earth alkali metal halides, sulfates, nitrates, carbonates, borates, and phosphates, more preferably alkali metal halides, such as bromides or chlorides, e.g. sodium or potassium chloride. The tonicity builder is present in an amount sufficient to provide a tonicity in the final solution of the dosage regimen of 50 to 400 mosmole, most preferably 250 to 350 mosmole.

The pH regulator maintains the pH preferably in the range of 7.5 to 11.5, more preferably 8.0 to 10.0, most preferably 8.5 to 9.5. Said pH is maintained advantageously for at least a period of time of from 10 minutes to 24 hours, preferably 30 minutes to 8 hours, more preferably 1 hour to 6 hours or until a pH altering agent is added to the solution. The pH regulating agent is typically selected from inorganic or organic bases, preferably basic acetates, phosphates, borates, nitrates, sulfates, tartrates, lactates, carbonates, bicarbonates and mixtures thereof, more preferably basic phosphates, borates, citrates, tartrates, carbonates, bicarbonates and mixtures thereof. Typically, it is present in an amount of 0.001 % to 2 %, preferably 0.01% to 1 %. The pH altering agent may or may not be a part of the initial composition and, when not part of the initial composition, may be added after a specified period of time as set forth above. The pH altering agent is preferably selected from inorganic or organic acids, more preferably acidic acetates, phosphates, borates, nitrates, sulfates, citrates, tartrates, lactates, carbonates, and bicarbonates, most preferably acidic phosphates, borates, citrates, tartrates, carbonates and mixtures thereof. The pH altering agent is present in an amount, when part of the original composition, or added in an amount, when not, which is sufficient to overcome the pH regulating agent and reset the pH to that desired. A pH typically desired is a pH corresponding to the pH of the tear fluid, e.g. 6.5 to 7.5.

As stated above, the cleaner compositions do not contain any enzymes for the removal of protein.

Enzyme, as used in the present context, is intended to mean a catalytic biological entity capable of degrading protein.

The compositions of the present invention can be formulated in nearly any conventional manner that would be considered suitable by those of ordinary skill.

More specifically, the present invention is directed to the removal of protein, especially lysozyme and other proteins typically adhering to contact lenses, such as IgM, serum IgA, secretory IgA, IgG, lactoferrin, albumin, peroxidase and tear specific prealbumin, from contact lenses, especially soft contact lenses. In the contact lens field lysozyme (found in tear fluid), its deposition on contact lenses and subsequent cleaning of lenses so fouled is a continuous and thorny problem. Most contact lenses have polymeric matrix structures which contain multiple negative sites at neutral or approximately neutral pH. Lysozyme, and a significant number of other proteins, have an isoelectric point above the usual range of pH in the cleaners used with contact lenses. Specifically, lysozyme has an isoelectric point of about 10-11. Below the isoelectric point, the protein has substantial positive charge and hence is attracted to the lens matrix. Enzymes, which cleave the protein into smaller pieces but do not account for the attractive ionic forces, only permit the fragment to permeate still further into the lens matrix, making cleaning even harder.

In the instant invention, the pH of the cleaner is maintained in the range of 7.5 to 11.5, which is near the isoelectric point. Since the lysozyme will have significantly less positive charge and may have a net negative charge the attractive force of the lysozyme for the lens will diminish or even be replaced by a weak repulsive force. In this way the protein contaminant on the lens can be readily removed.

In addition, the present invention offers advantages over the enzyme cleaners. First, the present invention does not fragment the adhered protein so that further migration of protein into the matrix is not increased. Additionally, as enzymes are themselves proteins, with the enzyme cleaners, there is risk of the lens having residual enzyme present even after careful rinsing. The present invention has no added enzyme and therefore is free of this problem. Still further, as enzymes continue to remain active after disposal, the present, enzyme free, formulations are much more environmentally elegant and suitable than prior enzyme containing compositions.

Still more specifically, the present invention is directed to the use of compositions for cleaning protein deposits from contact lenses in the absence of enzymes and in the absence of a protein dissolving effective amount of a surfactant consisting of a 250 to 350 mosmole solution at pH of 7.5 to 11.5 by basic borate or phosphate buffer and sodium chloride.

A means for creating cleaning solution according to the invention is e.g. a solid pH-controlled contact lens cleaner dosage form consisting of (a) a sufficient amount of tonicity builder to render ophthalmically acceptable an amount of water which is sufficient to cover a contact lens; and (b) a pH regulator sufficient to provide and maintain a pH of 7.5 to 11.5 in said amount of water; in the substantial absence of a protein-digesting enzyme. Said solid dosage form may be in the form of tablets, capsules, powder, granules or pellets, tablets being preferred, which can be formulated from or with conventional excipients, as known in the art.

In a further embodiment, the use of a one step system is contemplated wherein e.g. an outer shell of a solid dosage form, e.g. of a tablet, consists of the ingredients for carrying out the cleaning according to the instant invention, and a delayed release core comprises a pH altering agent adjusting the pH of the cleaning solution into a tolerable range from which the lens, without rinsing or with minimal rinsing, can be placed directly on the users eye without significant irritation.

In such case the solid phase pH-controlled cleaner composition, e.g. contact lens cleaner composition, consists of a delayed release component and an immediate release component, said immediate release component dissolving in an aqueous environment to form a first solution, said immediate release component consisting in the substantial absence of a protein digesting enzyme and in the substantial absence of a protein dissolving amount of a surfactant, of a pH regulator in an effective buffering amount, and a tonicity builder in a sufficient amount, such that upon complete dissolution of the immediate release component of the solid cleaner composition, the resulting solution has a tonicity of from 200 to 400 mosmol, preferably 200 to 300 mosmol, and a pH of 7.5 to 11.5; said delayed release component dissolving in said first solution from 10 minutes to 24 hours after said solid cleaner composition is introduced into said aqueous environment, said delayed release component comprising ophthalmically acceptable coating agents such as hydroxypropylmethylcellulose phthalate, sucrose, gelatin, methylcellulose, polyethylene glycol, maleic acid copolymers, methacrylic acid polymers, cellulose acetate phthalate ethylcellulose or mixtures thereof, and a pH altering agent, whereby said first solution is converted into a second solution, said second solution having a pH of 6.5 to 7.5, and having a preferred tonicity of 250 to 350 mosmol.

In this embodiment, the cleaner solution is the result of e.g. a coating on a core dissolving, and after a specified time in which the lens resides in such solution, the core dissolves, whereby the cleaner solution is transformed in situ into a type of soaking solution. In such an embodiment, at minimum, the pH is returned to one from which with minimal rinsing direct administration to the eye would be suitable.

The method according to the present invention consists of contacting a proteinacous deposit containing polymeric material, especially a contact lens, with a solution as hereinbefore described, wherein the pH is between 7.5 and 11.5, preferably 8.2 and 10, more preferably 8.5 to 9.5, for a period of up to 24 hours, peferably 10 minutes to 12 hours, more preferably 30 minutes to 8 hours, most preferably 1 hour to 6 hours, in the substantial absence of a proteinaceous deposit digesting enzyme, and removing the contact lens from such solution. The tonicity of said solution is preferably ophthalmically acceptable. If desired the so cleaned lens may then be rinsed or placed into a soaking solution.

The instant invention will be more fully understood by reference to the following illustrative, but non-limiting Examples.

Example 1: Ten Vifilcon A® lenses are soaked for 18-20 hours in a 0.12 % lysozyme solution containing 0.7 % sodium chloride, 0.58 % boric acid, and 0.05 % sodium borate decahydrate, adjusted to pH 7.2 with dilute HCl or dilute NaOH as needed. Lysozyme deposition is determined by absorbance measurement at 280 nm. The lenses are then placed in one of five buffered saline solutions (as set forth in Table I) for 2 hours, rinsed in saline, and absorbance at 280 nm is measured again and is reported in Table I (hereinafter).

Example 2 (Comparative): The procedure of example 1 is followed except that enzymatic cleaners designated in Table II, having the given pH values, after tablet dissolution are used instead of the present invention solution (Table II follows Table I).

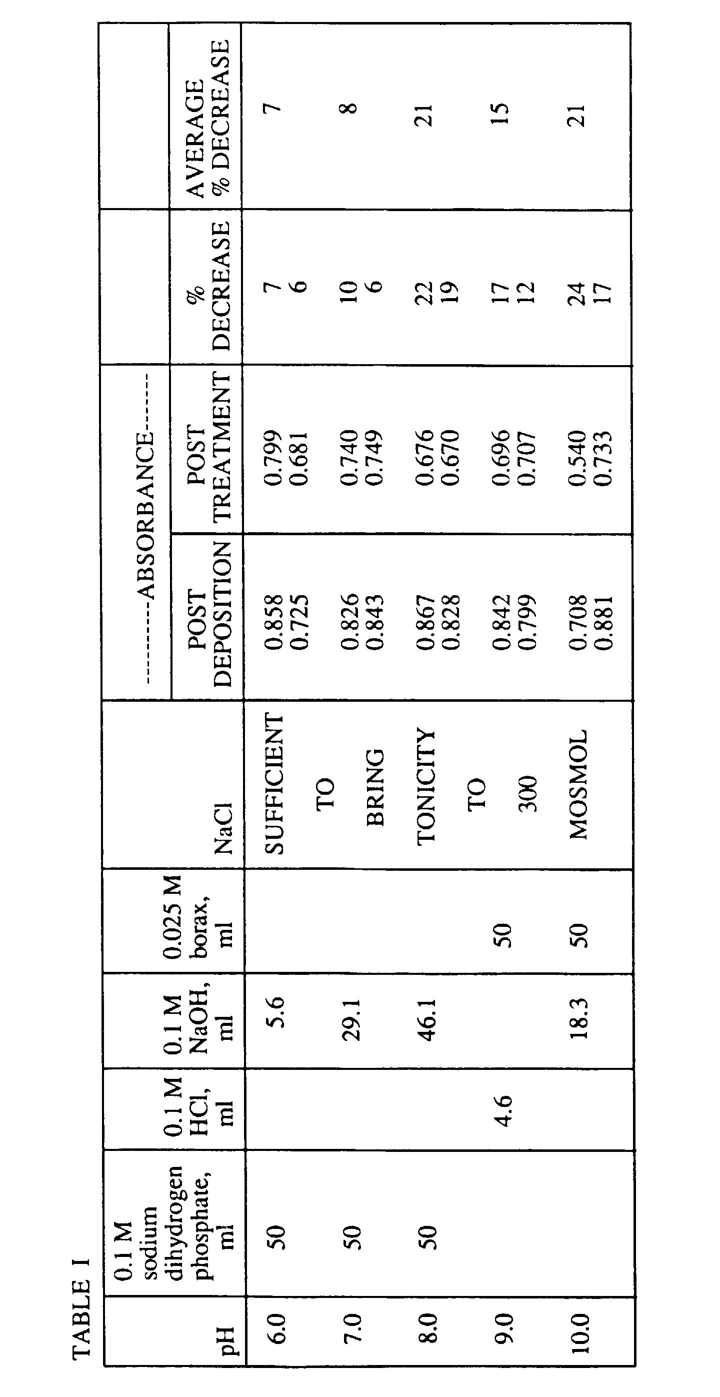

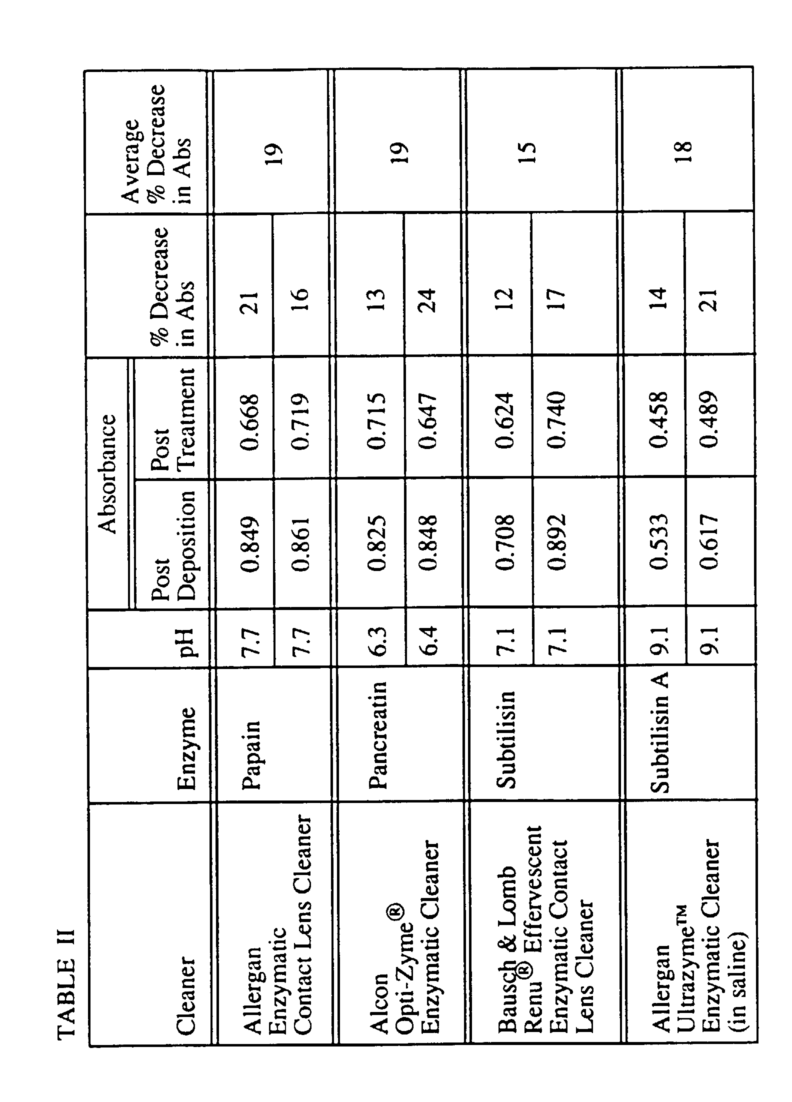

Example 3 (Comparative): The procedure of example 1 is followed except that Bausch & Lomb's ReNu™ Effervescent Enzymatic Cleaner containing the enzyme subtilisin is added to the instant invention solution, in the same concentration as in example 2. This eliminates any confusion due to other materials in example 2 which are dissimilar to those in example 1. The results are shown in Table III.

**TABLE III**

| | Absorbance | | | Average % Decrease in Abs |
|---|---|---|---|---|
| pH | Post Deposition | Post Treatment | % Decrease in Abs | |
| 6 | 0.750 | 0.677 | 10 | 8 |
| | 0.749 | 0.701 | 6 | |
| 7 | 0.828 | 0.763 | 8 | 10 |
| | 0.736 | 0.649 | 12 | |
| 8 | 0.674 | 0.608 | 10 | 13 |
| | 0.949 | 0.794 | 16 | |
| 9 | 0.582 | 0.514 | 12 | 15 |
| | 0.961 | 0.785 | 18 | |
| 10 | 0.653 | 0.541 | 17 | 14 |
| | 0.700 | 0.629 | 10 | |

## Claims

1. Use of a pH-controlled contact lens cleaner composition for removing proteinaceous deposits from a contact lens in the substantial absence of a proteinaceous deposit digesting enzyme and in the substantial absence of a protein dissolving amount of a surfactant, wherein said cleaner composition consists of (a) water, (b) a tonicity builder to render said composition ophthalmically acceptable, and (c) a pH regulator sufficient to provide and maintain a pH of 7.5 to 11.5.

2. Use of a solid pH-controlled contact lens cleaner composition for removing proteinaceous deposits from a contact lens in the substantial absence of a proteinaceous deposit digesting enzyme and in the substantial absence of a protein dissolving amount of a surfactant, wherein said solid cleaner composition consists of (a) a sufficient amount of tonicity builder to render ophthalmically acceptable an amount of water which is sufficient to cover a contact lens; and (b) a pH regulator sufficient to provide and maintain a pH of 7.5 to 11.5 in said amount of water.

3. Use of a solid phase pH-controlled contact lens cleaner composition for removing proteinaceous deposits from a contact lens in the substantial absence of a proteinaceous deposit digesting enzyme and in the substantial absence of a protein dissolving amount of a surfactant, wherein said cleaner composition consists of a delayed release component and an immediate release component according to claim 2, such that upon complete dissolution of the immediate release component of the solid cleaner composition, the resulting first solution has a tonicity of from 200 to 400 mosmol and a pH of 7.5 to 11.5; said delayed release component dissolving in said first solution from 10 minutes to 24 hours after said solid cleaner composition is introduced into said aqueous environment, said delayed release component comprising an ophthalmically acceptable coating agent, and a pH altering agent, whereby said first solution is converted into a second solution, said second solution having a pH of 6.5 to 7.5.

4. The use according to claim 1 or 2, wherein the tonicity builder is sodium chloride.

5. The use according to claim 1 or 2, wherein the pH-regulator is basic borate or phosphate.

6. The use according to claim 1 or 2, wherein the tonicity builder is sodium chloride in an amount to bring the tonicity of the composition to 250 to 350 mosmole and the pH-regulator is basic borate or phosphate in an amount to maintain the pH of the composition at 7.5 to 11.5.

7. The use according to claim 3, characterized in that the tonicity of the second solution is in the range of 250 to 350 mosmol.

8. The use according to claim 3, wherein the coating agent of the delayed release component is selected from hydroxypropylmethylcellulose phthalate, sucrose, gelatin, polyvinylpyrrolidone, methylcellulose, polyethylene glycol, maleic acid copolymers, methacrylic acid polymers, cellulose acetate phthalate ethylcellulose or mixtures thereof.

9. A method of removing proteinaceous deposits from a contact lens in the substantial absence of a proteinaceous deposit digesting enzyme and in the substantial absence of a protein dissolving amount of a surfactant, which method consists of contacting said contact lens having a proteinaceous deposit thereon with an aqueous solution according to claims 1-8 to free at least a significant portion of said proteinaceous deposit from said contact lens and removing said proteinaceous deposit reduced contact lens from said solution.

## Patentansprüche

1. Verwendung eines pH-gesteuerten Kontaktlinsen-Reinigungsmittels zum Entfernen von Proteinablagerungen von einer Kontaktlinse in wesentlicher Abwesenheit eines eine Proteinablagerung aufschließenden Enzyms und in wesentlicher Abwesenheit einer Protein lösenden Menge eines Tensids, wobei das Reinigungsmittel aus (a) Wasser, (b) einem Tonizitätsbuilder, um das Mittel ophthalmisch verträglich zu machen und (c) einem pH-Steuerungsmittel, ausreichend zur Bereitstellung und zum Beibehalten eines pH-Werts von 7,5 bis 11,5, besteht.

2. Verwendung eines festen, pH-gesteuerten Kontaktlinsen-Reinigungsmittels zur Entfernung von Proteinablagerungen von einer Konnaktlinse in wesentlicher Abwesenheit eines eine Proteinablagerung aufschließenden Enzyms und in wesentlicher Abwesenheit einer Protein lösenden Menge eines Tensids, wobei das feste Reinigungsmittel aus (a) einer ausreichenden Menge eines Tonizitätsbuilders, um eine zur Bedeckung der Kontaktlinsen ausreichende Menge Wasser ophthalmisch verträglich zu machen; und (b) einem pH-Steuerungsmittel, ausreichend zur Bereitstellung und zum Beibehalten eines pH-Werts von 7,5 bis 11,5 in der Wassermenge, besteht.

3. Verwendung eines pH-gesteuerten Kontaktlinsen-Festphasen-Reinigungsmittels zur Entfernung von Proteinablagerungen von einer Kontaktlinse in wesentlicher Abwesenheit eines eine Proteinablagerung aufschließenden Enzyms und in wesentlicher Abwesenheit einer Protein lösenden Menge eines Tensids, wobei das Reinigungsmittel aus einer Komponente mit verzögerter Freisetzung und einer Komponente mit unmittelbarer Freisetzung nach Anspruch 2 besteht, so daß nach vollständiger Auflösung der Komponente mit unmittelbarer Freisetzung des festen Reinigungsmittels die erhaltene erste Lösung eine Tonizität von 200 bis 400 mOsmol und einen pH-Wert von 7,5 bis 11,5 aufweist; wobei sich die Komponente mit verzögerter Freisetzung in der ersten Lösung 10 Minuten bis 24 Stunden, nachdem das feste Reinigungsmittel in die wässerige Umgebung eingeführt wurde, auflöst, wobei die Komponente mit verzögerter Freisetzung ein ophthalmisch verträgliches Beschichtungsmittel und ein pH-Wert änderndes Mittel umfaßt, wodurch die erste Lösung in eine zweite Lösung umgewandelt wird, wobei die zweite Lösung einen pH-Wert von 6,5 bis 7,5 aufweist.

4. Verwendung nach Anspruch 1 oder 2, wobei der Tonizitätsbuilder Natriumchlorid ist.

5. Verwendung nach Anspruch 1 oder 2, wobei das pH-Steuerungsmittel ein basisches Borat oder Phosphat ist.

6. Verwendung nach Anspruch 1 oder 2, wobei der Tonizitätsbuilder Natriumchlorid in einer Menge darstellt, um die Tonizität des Mittels auf 250 bis 350 mOsmol zu bringen und das pH-Steuerungsmittel basisches Borat oder Phosphat in einer Menge darstellt, um den pH-Wert des Mittels bei 7,5 bis 11,5 zu halten.

7. Verwendung nach Anspruch 3, dadurch gekennzeichnet, daß die Tonizität der zweiten Lösung im Bereich von 250 bis 350 mOsmol liegt.

8. Verwendung nach Anspruch 3, wobei das Beschichtungsmittel der Komponente mit verzögerter Freisetzung ausgewählt ist aus Hydroxypropylmethylcellulosephthalat, Saccharose, Gelatine, Polyvinylpyrrolidon, Methylcellulose, Polyethylenglycol, Maleinsäure-Copolymeren, Methacrylsäurepolymeren, Celluloseacetatphthalat, Ethylcellulose oder Gemischen davon.

9. Verfahren zum Entfernen von Proteinablagerungen von einer Kontaktlinse in wesentlicher Abwesenheit eines eine Proteinablagerung aufschließenden Enzyms und in wesentlicher Abwesenheit einer Protein lösenden Menge eines Tensids, wobei das Verfahren im Inkontaktbringen der eine Proteinablagerung darauf aufweisenden Linse mit einer wässerigen Lösung nach Ansprüchen 1 bis 8, damit von der Kontaktlinse mindestens ein signifikanter Teil der Proteinablagerung freigesetzt wird und Entfernen der mit weniger Proteinablagerung behafteten Kontaktlinse aus der Lösung, besteht.

## Revendications

1. Utilisation d'une composition de nettoyage pour lentilles de contact à pH contrôlé pour enlever les dépôts protéiniques des lentilles en l'absence substantielle d'une enzyme digérant les dépôts protéiniques et en l'absence substantielle d'une quantité dissolvant les protéines d'un tensio-actif, où la composition de nettoyage comprend (a) de l'eau, (b) un générateur de tonicité pour rendre ladite composition acceptable du point de vue ophtalmique, et (c) un régulateur de pH suffisant pour donner et maintenir le pH entre 7,5 et 11,5.

2. Utilisation d'une composition de nettoyage solide pour lentilles de contact à pH contrôlé pour enlever les dépôts protéiniques des lentilles en l'absence substantielle d'une enzyme digérant les dépôts protéiniques et en l'absence substantielle d'une quantité dissolvant les protéines d'un tensio-actif, où ladite composition de nettoyage solide comprend (a) une quantité suffisante d'un générateur de tonicité pour que la quantité d'eau qui est suffisante pour couvrir les lentilles de contact soit rendue acceptable du point de vue ophtalmique; et (b) un régulateur de pH suffisant pour donner et maintenir le pH entre 7,5 et 11,5 dans ladite quantité d'eau.

3. Utilisation d'une composition de nettoyage en phase solide pour lentilles de contact à pH contrôlé pour enlever les dépôts protéiniques des lentilles en l'absence substantielle d'une enzyme digérant les dépôts protéiniques et en l'absence substantielle d' une quantité dissolvant les protéines d'un tensio-actif, où ladite composition de nettoyage comprenant un composant à libération retardée et un composant à libération immédiate selon la revendication 2, de sorte qu'après dissolution complète du composant à libération immédiate de la solution de nettoyage solide, la première solution résultante a une tonicité de 200 à 400 mosmoles et un pH compris entre 7,5 et 11,5; ledit composant à libération retardée se dissolvant dans la première solution en l'espace de 10 minutes à 24 heures après introduction de la composition de nettoyage solide dans l'environnement aqueux, le composant à libération retardée comprenant un agent d'enrobage acceptable du point de vue ophtalmique et un agent modifiant le pH, la première solution étant transformée en une seconde solution laquelle a un pH compris entre 6,5 et 7,5.

4. Utilisation selon la revendication 1 ou 2, selon laquelle le générateur de tonicité est le chlorure de sodium.

5. Utilisation selon la revendication 1 ou 2, selon laquelle le régulateur du pH est un borate ou un phosphate basique.

6. Utilisation selon la revendication 1 ou 2, selon laquelle le générateur de tonicité est le chlorure de sodium en une quantité destinée à amener la tonicité de la composition entre 250 et 350 mosmoles et le régulateur de pH est un borate ou un phosphate basique en une quantité destinée à maintenir le pH de la composition entre 7,5 et 11,5.

7. Utilisation selon la revendication 3, caractérisée en ce que la tonicité de la seconde solution est comprise entre 250 et 300 mosmoles.

8. Utilisation selon la revendication 3, selon laquelle l'agent d'enrobage du composant à libération retardée est choisi parmi le phtalalate d'hydroxypropylméthylcellulose, le saccharose, la gélatine, la polyvinylpyrrolidone, la méthylcellulose, le polyéthylèneglycol, les copolymères de l'acide maléique, les polymères de l'acide méthacrylique, le phtalate d'acétate de cellulose, l'éthylcellulose ou leurs mélanges.

9. Un procédé destiné à enlever les dépôts protéiniques des lentilles de contact en l'absence substantielle d'une enzyme digérant les dépôts protéiniques et en l'absence substantielle d'une quantité dissolvant les protéines d'un tensio-actif, procédé selon lequel des lentilles ayant un dépôt protéinique sont mises en contact avec une solution aqueuse selon les revendications 1 à 8 pour enlever au moins une proportion importante du dépôt protéinique des lentilles de contact, et retirer de ladite solution les lentilles de contact ayant un dépôt protéinique réduit.
